# EUROPEAN PATENT SPECIFICATION

(11) **EP 1 728 481 B1**
(45) Date of publication and mention of the grant of the patent: **19.03.2008**
(21) Application number: 06380126.0
(22) Date of filing: 24.05.2006
(51) Int. Cl.: A61B 18/12

(54) **Transformer for capacitive/resistive conversion of an electrosurgical RF output**
Transformator zur kapazitiv/resistiven Konvertierung eines elektochirurgischen RF-Ausgangs
Transformateur pour conversion capacitive/résistive d'une sortie RF électrochirurgicale

(30) Priority: 02.06.2005 ES 200501260 U
(43) Date of publication of application: 06.12.2006
(73) Proprietor: INDIBA, S.A., E-08013 Barcelona (ES)
(72) Inventor: Lario Garcia, Javier c/o Indiba S.A., 08013 Barcelona (ES)
(74) Representative: Pastells Teixido, Manuel

(56) References cited:
- EP-A- 0 893 140
- WO-A-97/17029
- GB-A- 2 146 534
- US-A- 4 802 470

## Description

### OBJECT OF THE INVENTION

A convertive device being applicable to capacitive transfer electronic recovery apparatuses for them to be used as a resistive system.

### FIELD OF THE INVENTION

This device is designed to be used with the medical therapy apparatuses comprising a general high-frequency, electronic circuit and a tool being made up by an active electrode, these apparatuses being fit to operate in a capacitive manner.

### BACKGROUND OF THE INVENTION

This very Applicant is the owner of the Spanish patent of invention no. 9701641 (ES-2139 507) having as its object an electrotherapy apparatus being operable for generating an electric current having a high relative frequency and being applicable to the human body by means of a capacitive, active electrode (being made of metal and having an electrically insulating covering) and a neutral electrode, said electrodes being connected to the secondary of the transformer of the output stage of the corresponding circuit, an intermediary terminal being connected to said secondary for the connection of a resistive, active electrode (being made of metal without an insulating covering).

A device according to the preamble of independent claim 1 is known from GB 2 146 534.

### SUMMARY OF THE INVENTION

This invention has as its object a convertive device that when being applied to the capacitive transfer electronic recovery apparatuses transforms them into apparatuses being fit to be used as a resistive system.

This convertive device is fully independent and can be connected and disconnected to and from the recovery apparatuses being used for carrying out a capacitive transfer in order to use them as such or to transform them into resistive transfer apparatuses.

A characterising feature of this convertive device lies in its comprising an output transformer with a primary and a secondary being arranged in a separate arrangement, the primary outputs being designed to be removably connected with the active pole and the neutral pole of the capacitive recovery apparatus, the active and neutral pads of the resistive system being connected to the outputs of the secondary.

These and other characterising features will be best made apparent by the following detailed description whose understanding will be made easier by the accompanying sheet of drawings showing a practical embodiment being cited only by way of example not limiting the scope of the present invention.

### DESCRIPTION OF THE DRAWINGS

In the drawings:

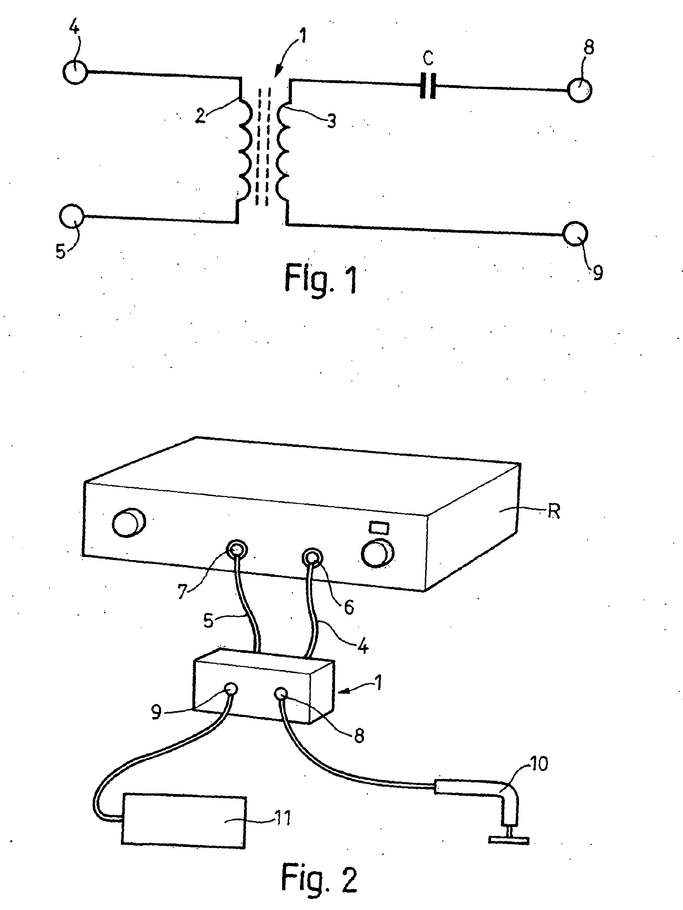

Fig. 1 illustrates the transformer corresponding to the convertive device being the object of the invention; and
Fig. 2 is a perspective view diagrammatically depicting the assembly of this convertive device being connected to a capacitive transfer recovery apparatus.

### DETAILED DESCRIPTION

According to the drawings this convertive device comprises an output transformer (1) with a primary (2) and a secondary (3) being arranged in a separate arrangement, the outputs (4) and (5) of the primary (2) being designed to be respectively connected with the active pole (6) and with the neutral pole (7) of the electronic recovery apparatus (R), the active pad (10) and the neutral pad (11) of the resistive system being connected to the outputs (8) and (9) of the secondary (3) of the transformer, said active pad being made up by a metallic electrode, and said neutral pad being made up by a metallic plate.

In this convertive device an output transformer is used in which the primary and the secondary are arranged in a separate arrangement and have the impedances being adequate for the system to operate in a resistive manner.

## Claims

1. A convertive device (1, 2, 3) for transforming the output of a medical therapy apparatus (R) having a general high-frequency electronic circuit into an output suitable for driving a resistive system, said resistive system having active (10) and neutral pads (11), the convertive device comprising an output transformer (1) with a primary (2) and a secondary coil (3) being arranged in a separate arrangement, the outputs (4, 5) of the primary (2) being designed to be connected with the poles (6, 7) of said medical therapy apparatus (R), the active pad (10) and the neutral pad (11) of the resistive system being connected to the outputs (8, 9) of the secondary coil (3) of the transformer, **characterised in that** the convertive device (1, 2, 3) is further adapted to transform the active (6) and neutral (7) pole outputs of said medical therapy apparatus (R) having a general high-frequency electronic circuit further adapted to drive a capacitive system, said capacitive system having a neutral electrode and an insulated active electrode, into an output suitable for driving said resistive system.

## Patentansprüche

1. Wandlervorrichtung (1, 2, 3) zum Umsetzen der Ausgabe eines medizinischen Therapiegeräts (R), das einen allgemeinen, elektronischen Hochfrequenzschaltkreis hat, in eine zum Betreiben eines resistiven Systems geeignete Ausgabe, wobei das vorgenannte resistive System eine aktive Elektrode (10) und eine neutrale Elektrode (11) hat, wobei die Wandlervorrichtung einen Ausgabewandler (1) mit einer primären Wicklung (2) und einer sekundären Wicklung (3) unfaßt, wobei diese letzteren in getrennter Anordnung angeordnet sind, wobei die Ausgänge (4, 5) der primären Wicklung dazu bestimmt sind, mit den Polen (6, 7) des vorgenannten medizinischen Therapiegeräts (R) verbunden zu werden, wobei die aktive Elektrode (10) und die neutrale Elektrode (11) des resistiven Systems mit den Ausgängen (8, 9) der sekundären Wicklung (3) des Wandlers verbunden sind; **dadurch gekennzeichnet, daß** die Wandlervorrichtung (1, 2, 3) außerden dazu adaptiert ist, die Ausgaben des aktiven Pols (6) und des neutralen Pols (7) des vorgenannten medizinischen Therapiegeräts (R) in eine zum Betreiben des vorgenannten resistiven Systems geeignete Ausgabe umzusetzen, wobei das vorgenannte medizinische Therapiegerät einen allgemeinen, elektronischen Hochfrequenzschaltkreis hat, der außerden dazu adaptiert ist, ein kapazitives System zu betreiben, wobei das vorgenannte kapazitive System eine neutrale Elektrode und eine isolierte aktive Elektrode hat.

## Revendications

1. Dispositif conversif (1, 2, 3) pour transformer la sortie d'un appareil de thérapie médicale (R) ayant un circuit électronique haute fréquence général en une sortie appropriée pour opérer un système résistif, ledit système résistif ayant des électrodes active (10) et neutre (11), le dispositif conversif comprenant un transformateur de sortie (1) avec une bobine primaire (2) et une bobine secondaire (3) étant disposées en agencement séparé, les sorties (4, 5) de la primaire (2) étant destinées à être connectés aux pôles (6, 7) dudit appareil de thérapie médicale (R), l'électrode active (10) et l'électrode neutre (11) du système résistif étant connectées aux sorties (8, 9) de la bobine secondaire (3) du transformateur, **caractérisé en ce que** le dispositif conversif (1, 2, 3) est en plus adapté pour transformer les sorties des pôles actif (6) et neutre (7) dudit appareil de thérapie médicale (R) ayant un circuit électronique haute fréquence général adapté pour opérer un système capacitif, ledit système capacitif ayant une électrode neutre et une électrode active isolée, en une sortie appropriée pour opérer ledit système résistif.
